# EUROPÄISCHE PATENTSCHRIFT

(11) **EP 0 882 692 B1**
(45) Veröffentlichungstag und Bekanntmachung des Hinweises auf die Patenterteilung: **07.11.2001**
(21) Anmeldenummer: 98108843.8
(22) Anmeldetag: 15.05.1998
(51) Int. Cl.: C07C 1/20

(54) **Verfahren zum Erzeugen von Ethylen, Propylen und wahlweise auch Butenisomeren aus Methanol und/oder Dimethylether**
Process for the preparation of ethylene, propylene and optionally isomers of butene from methanol and/or dimethyl ether
Procédé pour la préparation d'éthylène, de propylène et éventuellement d'isomères du butène à partir de méthanol et/ou de diméthyléther

(30) Priorität: 04.06.1997 DE 19723363
(43) Veröffentlichungstag der Anmeldung: 09.12.1998
(73) Patentinhaber: mg technologies ag, 60325 Frankfurt am Main (DE)
(72) Erfinder: Higman, Chistopher, 65824 Schwalbach (DE); König, Peter, Dr., 61348 Bad Homburg (DE); Möller, Friedrich-Wilhelm, Dr., 61381 Friedrichsdorf (DE); Holtmann, Hans-Dieter, Dr., 59199 Boenen (DE); Koss, Ulrich, 64291 Darmstadt (DE)
(74) Vertreter: Revesz, Veronika

(56) Entgegenhaltungen:
- EP-A- 0 074 075
- EP-A- 0 090 232
- EP-A- 0 448 000

## Beschreibung

Die Erfindung betrifft ein Verfahren zum Erzeugen von niederen Olefinen mit 2 und 3 C-Atomen im Molekül durch Umsetzen eines Methanol- und/oder Dimethyletherdampf und Wasserdampf enthaltenden Reaktionsgemisches in einem ersten Reaktor an einem ersten formselektiven Zeolith-Katalysator vom Pentasil-Typ bei Temperaturen im Katalysatorbereich von 280 bis 570 °C und einem Druck im Katalysatorbereich von 0,1 bis 1 bar, wobei man aus dem ersten Reaktor ein C₂- bis C₄-Olefine und Benzinkohlenwasserstoffe (C₅+) enthaltendes erstes Produktgemisch abzieht, welches gekühlt wird, welches man durch eine Trenneinrichtung führt und ein zweites, Ethylen und Propylen enthaltendes Produktgemisch abzieht und getrennt davon einen an den Benzinkohlenwasserstoffen reichen C₅₊-Strom erhält, wobei man den C₅₊-Strom erhält, wobei man den C₅₊-Strom verdampft und mit Wasserdampf mischt und dabei ein Gewichtsverhältnis H₂O:Kohlenwasserstoffe von 0,5 : 1 bis 3 : 1 einstellt, das wasserdampfhaltige Gemisch wird als Einsatzgemisch mit einer Eingangstemperatur von 380 bis 700 °C in einen zweiten Reaktor geleitet, der einen zweiten formselektiven Zeolith-Katalysator vom Pentasil-Typ enthält.

Ein solches Verfahren ist aus EP-A-0074075 bekannt, wobei man bevorzugt mit Borosilikat-Zeolithen als Katalysatoren arbeitet. Nach dem zweiten Reaktor erfolgt eine zweite Trennung, um Aromaten zu entfernen.

Ein Verfahren mit einem Reaktor ist aus EP 0 448 000 B 1 bekannt. Hierbei erzeugt man im ersten Reaktor, der als Röhrenreaktor ausgestaltet ist, ein Olefingemisch mit mindestens 5 Gew. -% Ethylen, mindestens 35 Gew. -% Propylen und höchstens 30 Gew. -% Butylen, bezogen auf die Gesamtkohlenwasserstoffe.

Der Erfindung liegt die Aufgabe zugrunde, auf wirtschaftliche Weise die Ausbeute an Ethylen und Propylen und wahlweise auch Butenisomeren im Produkt erheblich zu steigern. Erfindungsgemäß wird die Aufgabe beim eingangs genannten Verfahren durch die im Patentanspruch 1 genannte Merkmalskombination gelöst, wobei man mit formselektiven Alumosilikat-Zeolithen vom Pentasil-Typ arbeitet.

In der Trenneinrichtung kann man wahlweise dafür sorgen, dass das zweite Produktgemisch Butenisomere (C₄) zusätzlich enthält oder mehr oder weniger frei davon ist. Die separat abgetrennten Butenisomere können einerseits als weiteres Produkt verwertet oder zusammen mit dem C₅₊-Strom weiterverarbeitet werden.

Die Umsetzung im ersten Reaktor, der als Röhrenreaktor ausgestaltet ist, ist ausführlich z. B. in EP 0 448 000 B1 beschrieben. Hierbei geht man von Methanol oder auch Dimethylether aus, wobei das dem Röhrenreaktor zugeführte Reaktionsgemisch üblicherweise ein Gewichtsverhältnis Wasser/Methanol oder Wasser/Methanoläquivalente von 0,1 : 1 bis 1,5 : 1 aufweist. Ein "Methanoläquivalent" entspricht nach der Gleichung 2 CH₃OH ----> CH₃-O-CH₃+H₂O einem halben Mol Dimethylether (DME). Das nach der vorstehenden Gleichung gebildete Wasser wird im zuvor genannten Gewichtsverhältnis im Reaktionsgemisch nicht berücksichtigt. Das Verhältnis zwischen' Methanoldampf und Dimethyletherdampf kann in weiten Grenzen variieren, d. h. es können im Extremfall 100% Methanol oder aber 100 % DME zusammen mit Wasserdampf in den Röhrenreaktor geleitet werden. Es kann vorteilhaft sein, Methanol zunächst in einem Vorreaktor an einem sauren Katalysator ganz oder teilweise zu DME umzuwandeln, wodurch die Reaktionswärme bei der Umsetzung im Röhrenreaktor niedriger als bei Verwenden eines hohen Methanolanteils ist.

Anstelle des Röhrenreaktors kann man als ersten Reaktor auch einen Wirbelschicht-Reaktor einsetzen, wobei das Wirbelbett mit Kühlelementen zum indirekten Kühlen des körnigen ersten Katalysators versehen ist. Das Reaktionsgemisch dient hierbei der Fluidisierung des Katalysators. Die Wirbelschicht kann als stationäre oder zirkulierende Wirbelschicht ausgestaltet sein.

Der für die Umsetzung im ersten und zweiten Reaktor geeigneter Zeolith-Katalysator ist ebenfalls in EP 0 448 000 B1 beschrieben. Es handelt sich hierbei um einen Protonen enthaltenden Katalysator vom Pentasil-Typ mit einem Alkaligehalt von weniger als 380 ppm und vorzugsweise weniger als 200 ppm. Dieser Katalysator weist einen ZnO-Gehalt von weniger als 0,1 Gew.-%, einen CdO-Gehalt von weniger als 0,1 Gew.-%, eine BET-Oberfläche von 300 bis 600 m²/g und ein Porenvolumen (nach der Quecksilberporosymmetrie bestimmt) von 0,3 bis 0,8 cm³/g auf. Vorzugsweise liegt der Druck im Bereich dieses Katalysators bei höchstens 0,9 bar und vorzugsweise im Bereich von 0,2 bis 0,7 bar.

Das aus dem ersten Reaktor kommende Gemisch, das hier als erstes Produktgemisch bezeichnet wird, enthält C₂- bis C₄-Olefine und daneben vor allem gesättigte Kohlenwasserstoffe (C₅₊) sowie in kleineren Mengen weitere Komponenten wie Aromaten und Naphthene. Zusammen mit dem zurückgeführten dritten Produktgemisch, welches aus dem zweiten Reaktor kommt, führt man das gekühlte erste Produktgemisch durch eine Trenneinrichtung. Die Trenneinrichtung kann in an sich bekannter Weise destillativ, adsorptiv, thermisch oder mittels Membranen arbeiten, auch können diese für die Gastrennung oder die Gas-Flüssigkeits-Trennung bekannten Verfahrensmaßnahmen kombiniert angewandt werden. Aus der Trenneinrichtung zieht man zunächst ein zweites, Ethylen und Propylen enthaltendes Produktgemisch ab, welches wahlweise auch Butenisomere enthalten kann.

Die Trenneinrichtung liefert ferner einen an den Benzinkohlenwasserstoffen reichen C₅₊-Strom, den man in Dampfform, gemischt mit Wasserdampf, in den zweiten Reaktor leitet. Hierbei sorgt man für ein Gewichtsverhältnis H₂O : Kohlenwasserstoffe von 0,5 : 1 bis 3 : 1 und eine Temperatur am Eingang des zweiten Reaktors von 380 bis 700°C und vorzugsweise 400 bis 600°C.

Es ist ferner möglich, die Trenneinrichtung so auszugestalten, daß man einen C₄-Olefine enthaltenden Partialstrom gewinnt, den man als weiteren Produktstrom abzieht oder aber ganz oder teilweise zusammen mit dem C₅₊-Strom in den zweiten Reaktor leitet. Dieser Partialstrom kann neben C₄-Olefinen auch noch einen gewissen Anteil an C₃-Olefinen und eventuell auch C₂-Olefinen enthalten.

Man arbeitet im zweiten Reaktor bevorzugt bei einem Druck von 0,2 bis 3 bar und zumeist bei 0,6 bis 1,5 bar. Ferner empfiehlt es sich, dafür zu sorgen, daß man dem zweiten Reaktor ein Gemisch zuführt, das möglichst frei von Komponenten ist, die dreifache C-C-Bindungen oder konjugierte Doppelbindungen haben, da sie zu Kohlenstoffablagerungen und so zum vorzeitigen Desaktivieren des Katalysators führen können.

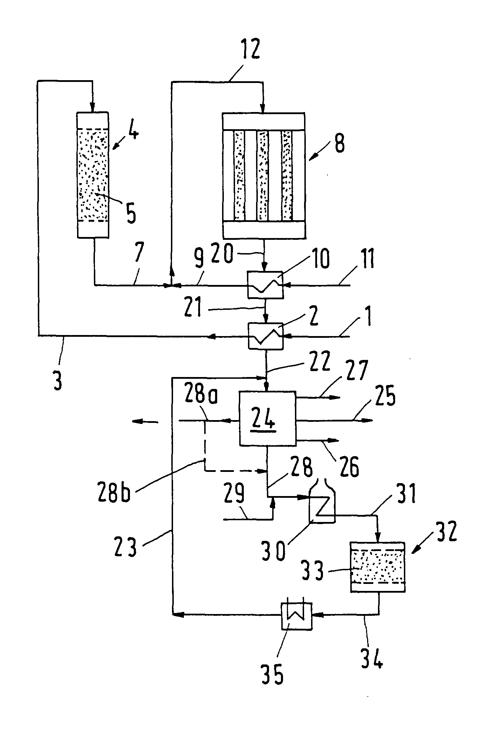

Ausgestaltungsmöglichkeiten des Verfahrens werden mit Hilfe der Zeichnung erläutert. Die Zeichnung zeigt ein Fließschema des Verfahrens.

Methanol wird in der Leitung (1) herangeführt, im Wärmeaustauscher (2) auf Temperaturen von etwa 250 bis 350°C erhitzt und dadurch verdampft. In der Leitung (3) führt man den Methanoldampf zu einem Vorreaktor (4), der eine Schüttung (5) aus einem körnigen Dehydratisierungskatalysator enthält. Als Katalysator kann z. B. Aluminiumoxid verwendet werden, wie es im US-Patent 4 058 576 beschrieben ist. Im Vorreaktor (4) wird ein Teil des Methanols zu DME und Wasser umgewandelt, das entstehende Gemisch zieht man in der Leitung (7) ab. Durch die Umsetzung im Vorreaktor (4) wird die Wärmeerzeugung im nachfolgenden Röhrenreaktor (8) abgemildert. Deshalb ist der Vorreaktor (4) nicht in allen Fällen erforderlich.

Dem Gemisch der Leitung (7) gibt man durch die Leitung (9) eine dosierte Menge an Wasserdampf zu, der vom Wärmeaustauscher (10) kommt. Kühlwasser zum Erzeugen des Wasserdampfs wird dem Wärmeaustauscher (10) in der Leitung (11) zugeführt. Dem Röhrenreaktor (8) gibt man durch die Leitung (12) ein Gemisch aus Methanol, DME und Wasserdampf auf, dessen Temperatur im Bereich von etwa 250 bis 450°C liegt. Das Gemisch der Leitung (12) weist ein Wasser/Methanoläquivalent-Gewichtsverhältnis von 0,1 : 1 bis 1,5 : 1 und vorzugsweise 0,2 : 1 bis 1 : 1 auf, dabei ist ein Mol DME = 2 Mol Methanoläquivalent. Im Röhrenreaktor (8) ist der Katalysator in zahlreichen Röhren angeordnet, die üblicherweise eine Länge von 1 bis 5 m und einen inneren Durchmesser von 20 bis 50 mm haben. Für die Umsetzung von Methanol und DME zu den gewünschten niederen Olefinen ist einerseits eine gute Kühlung des Katalysators und andererseits ein unteratmosphärischer Druck im Katalysatorbereich von 0,1 bis 0,9 bar und vorzugsweise 0,2 bis 0,7 bar zu empfehlen. Die indirekte Kühlung des Katalysators, der in den Röhren angeordnet ist, erfolgt bevorzugt durch ein Salzbad (vergleiche EP 0 448 000 B1). Als Alternative zum Röhrenreaktor (8) ist der Einsatz eines Wirbelschicht-Reaktors mit indirekter Kühlung des Wirbelbettes aus Katalysator-Granulat möglich.

Im Reaktor (8) liegen die Temperaturen im Katalysatorbereich bei 280 bis 570°C und vorzugsweise bei mindestens 300°C. Ein Produktgemisch, das hier als "erstes Produktgemisch" bezeichnet wird, verläßt den Reaktor (8) durch die Leitung (20), wird zunächst im Wärmeaustauscher (10) gekühlt, strömt dann durch die Leitung (21) zum zweiten Wärmeaustauscher (2) zur weiteren Kühlung und tritt dann durch die Leitung (22) zusammen mit dem Gemisch der Leitung (23) in die Trenneinrichtung (24) ein. Die Trenneinrichtung ist, wie oben erwähnt, in an sich bekannter Weise ausgestaltet. Man zieht aus ihr durch die Leitung (25) ein zweites Produktgemisch ab, welches hauptsächlich aus den erwünschten C₂- bis C₄-Olefinen besteht und üblicherweise einen Propylen-Gehalt von mindestens 40 Gew.-% aufweist. Eine wasserhaltige Fraktion zieht man in der Leitung (26) ab, und eine gasförmige Fraktion wird in der Leitung (27) aus dem Verfahren entfernt. Es kann ferner zweckmäßig sein, in der Trenneinrichtung einen an C₄-Olefinen reichen Partialstrom (28a) zu erzeugen. Diesen Partialstrom kann man getrennt abführen oder aber ganz oder teilweise durch die gestrichelte Leitung (28b) dem Strom der Leitung (28) zumischen.

In der Trenneinrichtung (24) fällt ein C₅₊-Strom an, der vor allem aus Benzinkohlenwasserstoffen besteht und den man in der Leitung (28) abführt. Diesem Strom mischt man in der Leitung (29) in dosierter Menge Wasser zu und führt das Gemisch durch einen gefeuerten Erhitzer (30), um es dann in der Leitung (31) mit einer Temperatur von 380 bis 700°C und vorzugsweise 400 bis 600°C in den zweiten Reaktor (32) zu leiten. Dieser zweite Reaktor enthält die bereits beschriebene Schüttung aus einem körnigen, formselektiven Zeolith-Katalysator. Der Katalysator ist in der Lage, die herangeführten Olefine zu spalten und bevorzugt Moleküle mit 2,3 und 4 C-Atomen zu bilden. Auf diese Weise besteht der in der Leitung (34) abgezogene Strom, der hier als "drittes Produktgemisch" bezeichnet wird, vor allem aus Propylen, und der summierte Gehalt an Ethylen, Propylen und Butenisomeren beträgt mindestens 60 Gew.-% der olefinischen Bestandteile des in der Leitung (31) herangeführten Einsatzgemisches.

Die Umsetzung im Reaktor (32) erfolgt adiabatisch, so daß die Temperatur des umzusetzenden Gemisches beim Durchströmen der Schüttung (33) sinkt. Dadurch ist die Temperatur in der Leitung (34) um 20 bis 80°C und zumeist 30 bis 50°C niedriger als die Eingangstemperatur in der Leitung (31). Im Reaktor (32) werden 80 bis 100 Gew.-% der Olefine umgesetzt, die im Gemisch der Leitung (31) herangeführt werden.

Im Kühler (35) wird das Gemisch der Leitung (34) auf Temperaturen von etwa 50 bis 100°C gekühlt, und dieses Gemisch wird dann durch die Leitung (23) in der bereits beschriebenen Weise zur Trenneinrichtung (24) geführt.

### Beispiel:

Im Labormaßstab wird mit einer Apparatur gearbeitet, die prinzipiell der Zeichnung entspricht. Die folgenden Daten beziehen sich auf 1 kg Methanol, das mit 240°C in den Vorreaktor (4) eintritt. Der Reaktor (4) enthält einen Dehydratisierungskatalysator auf der Basis von Gamma-Aluminiumoxid, er wird pro kg mit 2 kg/h Methanol beaufschlagt. 67% des Methanols werden im Reaktor (4) zu Dimethylether und Wasser umgesetzt. Dem Gemisch der Leitung (7) gibt man 0,5 kg Wasserdampf zu und führt es mit einer Eintrittstemperatur von 440°C in den Röhrenreaktor (8). Eine Salzschmelze dient im Reaktor (8) als Kühlflüssigkeit, der Katalysator ist der in EP 0 448 000 B1 beschriebene Zeolith vom Pentasil-Typ mit einem Alkaligehalt von 100 ppm und einem ZnO- und CdO-Gehalt von jeweils 0,05 Gew.%, die BET-Oberfläche beträgt 400 m²/g und des Porenvolumens 0,5 cm³/g. Der Druck am Reaktor-Eintritt ist auf 0,5 bar und die Temperatur des Kühlmittels ist auf 440°C eingestellt. Pro kg Katalysator und pro Stunde werden dem Reaktor (8) 1,5 kg Gemisch aus der Leitung (12) zugeführt.

Die Trenneinrichtung (24) ist als üblicher 3-Phasen-Abscheider ausgeführt, man erhält ein wässriges Kondensat, eine flüssige organische Phase und eine Gasphase. Das Kondensat, das in einer Menge von 1,06 kg anfällt, enthält nur sehr geringe Mengen an Alkoholen und Ketonen, der Methanolumsatz ist größer als 99,9%. Die flüssige Phase und die Gasphase wiegen zusammen 0,44 kg, zusammengenommen bestehen sie aus

| | |
|---|---|
| C₁ - C₄ -Paraffine | 5,1 Gew.% |
| Ethylen | 6,8 Gew.% |
| Propylen | 44,4 Gew.% |
| Butenisomere | 23,9 Gew.% |
| Benzin | 19,8 Gew.% |

Die Gasphase wird auf 1,3 bar komprimiert und die dabei gebildete Flüssigkeit mit dem Benzin gemischt. Man erhält so 112 g Benzinfraktion (C₅₊) mit folgender Zusammensetzung:

| | |
|---|---|
| C ₅₊ - Paraffine | 35,1 Gew.% |
| Naphthene | 6,9 Gew.% |
| Aromaten | 8,0 Gew.% |
| Butene | 13,4 Gew.% |
| Pentene | 21,5 Gew.% |
| Hexene | 10,1 Gew.% |
| Heptene | 5,0 Gew.% |

Dieses C₅₊-Gemisch wird verdampft, mit 166 g Wasser versetzt und auf 500°C erhitzt in einen Schachtreaktor (32) geleitet. Der Schachtreaktor enthält den gleichen Katalysator wir der Reaktor (8), der Druck am Austritt des Reaktors (32) wird auf 1,3 bar eingestellt. Pro kg Kataylsator werden 1,5 kg/h C₅₊-Gemisch über den Katalysator geleitet. Die Temperatur des in der Leitung (34) abgezogenen Gemisches beträgt 468°C, der organische Anteil dieses Gemisches besteht aus

| | |
|---|---|
| Ethylen | 5,6 Gew.% |
| Propylen | 20,1 Gew.% |
| Butene | 11,5 Gew.% |
| Brenngas (C₄₋) | 5,1 Gew.% |
| Benzin (C₅₊) | 57,7 Gew.% |

## Patentansprüche

1. Verfahren zum Erzeugen von niederen Olefinen mit 2 und 3 C-Atomen im Molekül durch Umsetzen eines Methanol- und/oder Dimethyletherdampf und Wasserdampf enthaltenden Reaktionsgemisches in einem ersten Reaktor (8) an einem ersten formselektiven Zeolith-Katalysator vom Pentasil-Typ bei Temperaturen im Katalysatorbereich von 280 bis 570 °C und einem Druck im Katalysatorbereich von 0,1 bis 1 bar, wobei man aus dem ersten Reaktor ein C₂- bis C₄-Olefine und Benzinkohlenwasserstoffe (C₅₊) enthaltendes erstes Produktgemisch abzieht, welches gekühlt wird, welches man durch eine Trenneinrichtung (24) führt und ein zweites, Ethylen und Propylen enthaltendes Produktgemisch abzieht und getrennt davon einen an den Benzinkohlenwasserstoffen reichen C₅₊-Strom erhält, wobei man den C₅₊-Strom verdampft und mit Wasserdampf mischt und dabei ein Gewichtsverhältnis H₂O : Kohlenwasserstoffe von 0,5 : 1 bis 3 : 1 einstellt, das wasserdampfhaltige Gemisch wird als Einsatzgemisch mit einer Eingangstemperatur von 380 bis 700 °C in einen zweiten Reaktor (32) geleitet, der einen zweiten formselektiven Zeolith-Katalysator vom Pentasil-Typ enthält, **dadurch gekennzeichnet, dass** der formselektive Zeolith-Katalysator vom Pentasil-Typ in beiden Reaktoren ein Alumosilikat-Zeolith ist, dass man den zweiten Reaktor (32) adiabatisch betreibt und darin 80 bis 100 Gew. -% der Olefine umsetzt, die im eintretenden Einsatzgemisch enthalten sind, dass man aus dem zweiten Reaktor ein drittes Produktgemisch abzieht, dessen summierter Gehalt an Propylen und Butenisomeren mindestens 50 Gew. -% der olefinischen Bestandteile des dem zweiten Reaktor zugeführten Einsatzgemisches beträgt, und dass man das dritte Produktgemisch kühlt und vollständig in die Trenneinrichtung (24) führt.

2. Verfahren nach Anspruch 1, **dadurch gekennzeichnet, daß** man in der Trenneinrichtung einen C₄ - Olefine enthaltenden Partialstrom abtrennt.

3. Verfahren nach Anspruch 1 oder 2, **dadurch gekennzeichnet, daß** der erste Reaktor ein Röhrenreaktor ist.

4. Verfahren nach Anspruch 1 oder einem der folgenden, **dadurch gekennzeichnet, daß** man im zweiten Reaktor bei einem Druck von 0,2 bis 3 bar arbeitet.

5. Verfahren nach Anspruch 1 oder einem der folgenden, **dadurch gekennzeichnet, daß** das dem ersten Reaktor zugeführte Reaktionsgemisch ein Gewichtsverhältnis Wasser/Methanol oder Wasser/Methanoläquivalente von 0,1 : 1 bis 1,5 : 1 aufweist.

## Claims

1. A process of producing lower olefins with 2 and 3 C atoms in the molecule by converting a reaction mixture containing methanol and/or dimethyl ether vapor and steam in a first reactor (8) on a first form-selective zeolite catalyst of the pentasil type at temperatures in the range from 280 to 570°C in the vicinity of the catalyst and at a pressure in the range from 0.1 to 1 bar in the vicinity of the catalyst, wherein from the first reactor a first product mixture containing C₂ to C₄ olefins and gasoline hydrocarbons (C₅₊) is withdrawn, which product mixture is cooled, is passed through a separating means (24), and a second product mixture containing ethylene and propylene is withdrawn and separate therefrom a C₅₊ stream rich in gasoline hydrocarbons is obtained, wherein the C₅₊ stream is evaporated and mixed with steam and a weight ratio H₂O : hydrocarbons of 0.5:1 to 3:1 is adjusted, the steam-containing mixture is introduced as feed mixture with an inlet temperature of 380 to 700°C into a second reactor (32) which contains a second form-selective zeolite catalyst of the pentasil type, **characterized in that** in both reactors the form-selective zeolite catalyst of the pentasil type is an alumosilicate zeolite, that the second reactor (32) is operated adiabatically and 80 to 100 wt-% of the olefins contained in the entering feed mixture are converted therein, that from the second reactor a third product mixture is withdrawn, whose added content of propylene and butene isomers is at least 50 wt-% of the olefinic constituents of the feed mixture supplied to the second reactor, and that the third product mixture is cooled and completely introduced into the separating means (24).

2. The process as claimed in claim 1, **characterized in that** a partial stream containing C₄ olefins is separated in the separating means.

3. The process as claimed in claim 1 or 2, **characterized in that** the first reactor is a tubular reactor.

4. The process as claimed in claim 1 or any of the preceding claims, **characterized in that** in the second reactor a pressure of 0.2 to 3 bar is employed.

5. The process as claimed in claim 1 or any of the preceding claims, **characterized in that** the reaction mixture supplied to the first reactor has a weight ratio water/methanol or water/methanol equivalents of 0.1:1 to 1.5:1.

## Revendications

1. Procédé pour la préparation d'oléfines inférieures avec 2 et 3 atomes C dans la molécule en convertissant un mélange réactionnel contenant de la vapeur de méthanol et/ou de diméthyléther et de la vapeur d'eau, dans un premier réacteur (8) sur un premier catalyseur à zéolite de type pentasil de forme sélective, à des températures dans la plage de 280 à 570°C dans la région du catalyseur et à une pression dans la plage de 0,1 à 1 bar dans la région du catalyseur, dans lequel on extrait du premier réacteur un premier mélange de produit contenant des oléfines C₂ à C₄ et des hydrocarbures d'essence (C₅₊), qui est refroidi, que l'on passe à travers un dispositif de séparation (24), et on extrait un deuxième mélange de produit contenant de l'éthylène et du propylène, et séparément, on obtient un flux de C₅₊ riche en hydrocarbures d'essence, en évaporant le flux de C₅₊ et en le mélangeant à de la vapeur d'eau en réglant un rapport en poids H₂O : hydrocarbures de 0,5 : 1 à 3 : 1, le mélange, qui renferme de la vapeur d'eau, étant introduit en tant que mélange de mise en oeuvre avec une température d'entrée de 380 à 700°C dans un deuxième réacteur (32) qui contient un deuxième catalyseur à zéolite du type pentasil de forme sélective, **caractérisé en ce que** le catalyseur à zéolite de type pentasil de forme sélective est, dans les deux réacteurs, un zéolite de silicate d'aluminium, **en ce que** l'on fait fonctionner le deuxième réacteur (32) en mode adiabatique en y convertissant 80 à 100 % en poids des oléfines, qui sont contenues dans le mélange de mise en oeuvre introduit, **en ce que** l'on extrait du deuxième réacteur un troisième mélange de produit dont la teneur en propylène et en isomères de butène totalisée s'élève à au moins 50 % en poids des constituants oléfiniques du mélange de mise en oeuvre amené au deuxième réacteur, et **en ce qu'**on refroidit le troisième mélange de produit et qu'on l'amène complètement dans le dispositif de séparation (24).

2. Procédé selon la revendication 1, **caractérisé en ce qu'**on sépare dans le dispositif de séparation un flux partiel contenant des oléfines C₄.

3. Procédé selon la revendication 1 ou 2, **caractérisé en ce que** le premier réacteur est un réacteur tubulaire.

4. Procédé selon la revendication 1 ou selon l'une des suivantes, **caractérisé en ce que** dans le deuxième réacteur on emploie une pression de 0,2 à 3 bar.

5. Procédé selon la revendication 1 ou selon l'une des suivantes, **caractérisé en ce que** le mélange réactionnel amené au premier réacteur présente un rapport en poids eau/méthanol ou eau/équivalents de méthanol de 0,1 : 1 à 1,5 : 1.
